# EUROPÄISCHE PATENTANMELDUNG

(11) **EP 3 168 635 A2**
(43) Veröffentlichungstag der Anmeldung: **17.05.2017**
(21) Anmeldenummer: 16192506.0
(22) Anmeldetag: 06.10.2016
(51) Int. Cl.: G01R 33/3875

(54) **VERFAHREN ZUM ANSTEUERN EINER SHIMEINHEIT, STEUEREINHEIT UND MAGNETRESONANZGERÄT**

(30) Priorität: 10.11.2015 DE 102015222114
(71) Anmelder: Siemens Healthcare GmbH, 91052 Erlangen (DE)
(72) Erfinder: Dewdney, Andrew, 91077 Neunkirchen am Brand (DE); Krug, Andreas, 90762 Fürth (DE); Eberlein, Eva, 91083 Baiersdorf (DE)

(57) **Zusammenfassung**

Die Erfindung betrifft ein Verfahren zum Ansteuern einer Shimeinheit, eine Steuereinheit und ein Magnetresonanzgerät. Um eine verbesserte Ansteuerung einer Shimeinheit zu ermöglichen, wird vorgeschlagen, dass das erfindungsgemäße Verfahren zum Ansteuern einer Shimeinheit eines Magnetresonanzgeräts während einer Magnetresonanz-Bildgebung eines Untersuchungsobjekts folgende Verfahrensschritte umfasst:
- Bereitstellen einer Gradientenpulsform gemäß Vorgaben einer Magnetresonanz-Sequenz, welche zur Magnetresonanz-Bildgebung des Untersuchungsobjekts eingesetzt wird,
- Modifizieren der Gradientenpulsform,
- Erzeugen von Shimeinstellungen unter Verwendung der modifizierten Gradientenpulsform,
- Ansteuern der Shimeinheit unter Verwendung der Shimeinstellungen, wobei das Ansteuern der Shimeinheit während eines Ausspielens der Gradientenpulsform mittels einer Gradientenspuleneinheit des Magnetresonanzgeräts erfolgt.

## Beschreibung

Die Erfindung betrifft ein Verfahren zum Ansteuern einer Shimeinheit, Steuereinheit und Magnetresonanzgerät.

In einem Magnetresonanzgerät, auch Magnetresonanztomographiesystem genannt, wird üblicherweise der zu untersuchende Körper einer Untersuchungsperson, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, beispielsweise Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Für eine bestimmte Messung ist daher eine bestimmte Magnetresonanz-Sequenz, auch Pulssequenz genannt, auszusenden, welche aus einer Folge von Hochfrequenz-Pulsen, beispielsweise Anregungspulsen und Refokussierungspulsen, sowie passend dazu koordiniert auszusendenden Gradientenpulsen mit bestimmten Gradientenpulsformen in verschiedenen Gradientenachsen entlang verschiedener Raumrichtungen besteht. Zeitlich passend hierzu werden Auslesefenster gesetzt, welche die Zeiträume vorgeben, in denen die induzierten Magnetresonanz-Signale erfasst werden.

Bei der Magnetresonanz-Bildgebung mittels eines Magnetresonanzgeräts ist die Homogenität eines Hauptmagnetfelds in einem Untersuchungsvolumen von großer Bedeutung. Bereits bei kleinen Abweichungen der Homogenität kann es zu großen Abweichungen in einer Frequenzverteilung der Kernspins kommen, so dass qualitativ minderwertige Magnetresonanz-Bilddaten aufgenommen werden. Um die Homogenität im Untersuchungsvolumen zu verbessern weist ein Magnetresonanzgerät typischerweise eine justierbare Shimeinheit auf. Insbesondere sind hierzu elektrische Shimspulen bekannt, die, mit verschiedenen Shimströmen angesteuert, verschiedene Kompensationsmagnetfelder erzeugen, um die Homogenität zu verbessern.

Das Aussenden von bestimmten von Vorgaben der Magnetresonanz-Sequenz festgelegten Gradientenpulsformen mittels einer Gradientenspuleneinheit des Magnetresonanzgeräts kann zu unerwünschten Wirbelstromfeldern in einem Aufnahmevolumen der Magnetresonanz-Sequenz führen. Der Betrieb der Gradientenspuleneinheit kann dabei Wirbelströme in allen elektrisch leitfähigen Komponenten des Magnetresonanzgeräts verursachen. Insbesondere in einer Abschirmung eines Magneten des Magnetresonanzgeräts, welche beispielsweise aus Aluminium besteht, können Wirbelströme besonders auftreten. Die im Aufnahmevolumen auftretenden Wirbelstromfelder können zu einem Qualitätsverlust der mittels der Magnetresonanz-Sequenz akquirierten Magnetresonanz-Bilder führen. So kann beispielsweise eine Fettunterdrückung bei bestimmten Magnetresonanz-Sequenzen durch die Wirbelstromfelder verschlechtert sein.

Zwar ist eine typische Gradientenspuleneinheit eines Magnetresonanzgeräts so ausgelegt, dass die durch den Betrieb der Gradientenspuleneinheit verursachten Wirbelströme und somit die Wirbelstromfelder in dem Aufnahmevolumen der Magnetresonanz-Sequenz minimiert werden. Allerdings können Fertigungstoleranzen bei der Herstellung der Gradientenspule zu einer Abweichung einer tatsächlichen Leiterführung in der Gradientenspule von der gewünschten Form führen. So können beispielsweise primäre Leitermuster in einer axialen Richtung gegenüber sekundären Leitermustern verschoben sein. Solch eine Verschiebung kann zu einem Verlust einer Symmetrie der Leiterführung in der Gradientenspuleneinheit und somit zu ganzzahligen Termen im Gradientenfeld und Wirbelstromfeld der Gradientenspuleneinheit führen. Gerade dünne Gradientenspuleneinheiten können sensitiver auf solch eine Verschiebung sein. Während die störenden Terme im Gradientenfeld typischerweise ignoriert werden können, so können die ganzzahligen Terme im Wirbelstromfeld der Gradientenspuleneinheit üblicherweise nicht ignoriert werden. Typische ganzzahlige Terme im Wirbelstromfeld der Gradientenspuleneinheit sind beispielsweise entlang der z-Achse (A(2,0) Terme), entlang der x-Achse (A(2,1) Terme) oder entlang der y-Achse (B(2,1) Terme) gerichtet.

Der Erfindung liegt die Aufgabe zu Grunde, eine verbesserte Kompensation von Wirbelströmen bei der Magnetresonanz-Bildgebung zu ermöglichen. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zum Ansteuern einer Shimeinheit eines Magnetresonanzgeräts während einer Magnetresonanz-Bildgebung eines Untersuchungsobjekts umfasst folgende Verfahrensschritte:
- Bereitstellen einer Gradientenpulsform gemäß Vorgaben einer Magnetresonanz-Sequenz, welche zur Magnetresonanz-Bildgebung des Untersuchungsobjekts eingesetzt wird,
- Modifizieren der Gradientenpulsform,
- Erzeugen von Shimeinstellungen unter Verwendung der modifizierten Gradientenpulsform,
- Ansteuern der Shimeinheit unter Verwendung der Shimeinstellungen, wobei das Ansteuern der Shimeinheit während eines Ausspielens der Gradientenpulsform mittels einer Gradientenspuleneinheit des Magnetresonanzgeräts erfolgt.

Das Untersuchungsobjekt kann ein Patient, ein gesunder Proband, ein Tier oder ein Phantom sein.

Die Shimeinheit ist typischerweise integriert in die Gradientenspuleneinheit des Magnetresonanzgeräts angeordnet. Teile der Shimeinheit oder die gesamte Shimeinheit können auch in Lokalspulen des Magnetresonanzgeräts angeordnet sein. Die Shimeinheit kann mehrere Shimkanäle bzw. Shimspulen umfassen. Die Shimkanäle bzw. Shimspulen der Shimeinheit können während der Magnetresonanz-Sequenz mit einem konstanten oder variablen Shimstrom belegt werden. Die Shimeinheit wird typischerweise von einer Shimsteuereinheit angesteuert, welche einen Shimstromverstärker aufweisen kann. Die Shimsteuereinheit kann die Shimeinheit mittels Shimeinstellungen ansteuern. Beispielsweise können die Shimeinstellungen eine, möglicherweise zeitabhängige, Stromverteilung der Shimströme in Shimspulen der Shimeinheit festlegen. Die Shim-Steuereinheit kann dann die Shimkanäle bzw. Shimspulen der Shimeinheit bei der Magnetresonanz-Bildgebung mit den durch die Shimeinstellungen festgelegten Strömen beaufschlagen.

Die Gradientenpulsform gibt insbesondere einen zeitlichen Verlauf einer Gradientenamplitude an, mit welcher die Gradientenspuleneinheit gemäß Vorgaben der Magnetresonanz-Sequenz angesteuert wird. Die Gradientenpulsform ist dabei insbesondere einer einzelnen Gradientenschaltung bzw. einem einzelnen Gradientenpuls oder auch mehreren Gradientenschaltungen bzw. Gradientenpulsen zugeordnet. Die Gradientenpulsform kann durch verschiedene Parameter, wie beispielsweise eine zeitliche Länge, eine maximale Amplitude, eine Flankensteilheit, definiert sein. Die Gradientenpulsform wird insbesondere von einer Steuereinheit des Magnetresonanzgeräts, insbesondere einer Gradientensteuereinheit, bereitgestellt bzw. erzeugt.

Das Modifizieren der Gradientenpulsform kann in der Gradientensteuereinheit oder in einer von der Gradientensteuereinheit separaten Einheit erfolgen. Insbesondere wird das Modifizieren der Gradientenpulsform von elektronischen Bauteilen oder digitalen Filtern durchgeführt. Das Modifizieren der Gradientenpulsform kann mittels eines digital und/oder elektronisch implementierten Modifizierungsalgorithmus erfolgen, welcher als Eingangsparameter die Gradientenpulsform und als Ausgangsparameter die modifizierte Gradientenpulsform aufweist.

Das Erzeugen der Shimeinstellungen unter Verwendung der modifizierten Gradientenpulsform kann ein direktes Ansteuern der Shimeinheit bzw. eines Shimverstärkers mit der modifizierten Gradientenpulsform umfassen. Alternativ oder zusätzlich kann die modifizierte Gradientenpulsform mittels einer, beispielsweise in einer Kalibrierungsmessung bestimmten, digital oder elektronisch implementierten Übertragungsfunktion in die Shimeinstellungen umgewandelt werden.

Die Shimeinheit wird insbesondere während des Ausspielens der Gradientenpulsform unter Verwendung der Shimeinstellungen derart angesteuert, dass zumindest während eines Teils des Zeitraums oder während des vollständigen Zeitraums des Ausspielens der Gradientenpulsform die gemäß der Shimeinstellungen festgelegte Stromverteilung der Shimströme in den Shimspulen der Shimeinheit fließt. Derart kann die Shimeinheit derart angesteuert werden, dass die Wirbelströme, welche durch das Ausspielen der Gradientenpulsform erzeugt werden, zumindest teilweise oder vollständig durch die mittels der Shimeinstellungen festgelegten Shimströme kompensiert werden. Das Ansteuern der Shimeinheit und das Ausspielen der Gradientenpulsform können derart zeitlich aufeinander abgestimmt, insbesondere mit einer gleichen Zeitverzögerung, erfolgen.

Die zeitliche Abstimmung des Ansteuerns der Shimeinheit und des Ausspielens der Gradientenpulsform kann dabei von besonderer Wichtigkeit sein. Die zeitliche Abstimmung kann dabei dadurch gewährleistet sein, dass das Erzeugen der Shimeinstellungen vorteilhafterweise automatisch durch das Bereitstellen und Modifizieren der Gradientenpulsform, welche ausgespielt werden soll, ausgelöst wird. Die Shimeinstellungen werden also insbesondere nicht unabhängig von der auszuspielenden Gradientenpulsform erzeugt, sondern können von der auszuspielenden Gradientenpulsform direkt abgeleitet werden. Es liegt also vorteilhafterweise ein direkter elektronisch und/oder digital implementierter Pfad vor, welcher aus einer zum Ausspielen bereitgestellten Gradientenpulsform mittels einer Modifikation der Gradientenpulsform Shimeinstellungen erzeugt, mittels welcher die Shimeinheit während des Ausspielens der Gradientenpulsform angesteuert werden kann. Besonders vorteilhaft können so durch das Ausspielen der Gradientenpulsform erzeugte Wirbelströme automatisch von der Shimeinheit kompensiert werden. Die zur Magnetresonanz-Bildgebung verwendete Magnetresonanz-Sequenz kann derart vorteilhafterweise unverändert bleiben, es ist also keine Anpassung der Magnetresonanz-Sequenz nötig.

Das vorgeschlagene Vorgehen kann derart zu einer besonders vorteilhaften und gut funktionierenden Unterdrückung von durch Gradientenschaltungen verursachten Wirbelströmen führen. Eine Qualität der in der Magnetresonanz-Bildgebung aufgenommenen Magnetresonanz-Bilddaten kann so erhöht werden bzw. Artefakte in den Magnetresonanz-Bilddaten können vermieden werden. Insbesondere können auch Gradientenspulen, welche in der Fertigungsprüfung höhere Abweichung von einer gewünschten Form aufweisen, trotzdem zum Kunden ausgeliefert werden und müssen nicht vernichtet werden.

Eine Ausführungsform sieht vor, dass das Modifizieren der Gradientenpulsform eine Bildung der Ableitung der Gradientenpulsform umfasst, wobei das Erzeugen der Shimeinstellungen unter Verwendung der Ableitung der Gradientenpulsform erfolgt. Das Bilden der Ableitung der Gradientenpulsform kann eine Differenzierung der Gradientenpulsform bzw. eine Berechnung von Veränderungen der Gradientenpulsform über die Zeit umfassen. Konstante Teile der Gradientenpulsform, beispielsweise Plateaus mit einer konstanten Amplitude, werden beim Bilden der Ableitung insbesondere unterdrückt. Gerade die steigenden bzw. fallenden Flanken der Gradientenpulsform treten beim Bilden der Ableitung in den Vordergrund. Die Bildung der Ableitung der Gradientenpulsform kann in einer dazu geeigneten Gradientensteuereinheit erfolgen, welche die Akzentuierung (Präemphase, pre-emphasis) und Deakzentuierung (Deemphase) der Gradientenpulsform für die Erzeugung der Shimeinstellungen weitergibt und Gleichstromkomponenten der Gradientenpuls bei der Weitergabe unterdrückt. Derart kann das vorgeschlagene Vorgehen bei dem Bereitstellen einer Gradientenpulsform für einen linearen Gradientenkanal der Gradientenspuleneinheit ein Ansteuern der Shimeinheit unter Verwendung von Shimeinstellungen, welche auf einer Ableitung der Gradientenpulsform basieren, umfassen. Dem Vorgehen gemäß dieser Ausführungsform liegt die Überlegung zugrunde, dass typischerweise Wirbelstromfelder während der steigenden bzw. fallenden Flanken der Gradientenpulsform erzeugt werden. Derart können mittels des Bildens der Ableitung der Gradientenpulsform Shimeinstellungen erzeugt werden, welche besonders vorteilhaft zur Kompensation der Wirbelstromfelder, welche durch das Ausspielen der Gradientenpulsform erzeugt werden, geeignet sind.

Eine Ausführungsform sieht vor, dass das Modifizieren der Gradientenpulsform und das Erzeugen der Shimeinstellungen unter Verwendung der modifizierten Gradientenpulsform derart erfolgen, dass Wirbelstrombeiträge zweiter Ordnung, welche während des Ausspielens der Gradientenpulsform mittels der Gradientenspuleneinheit auftreten, mittels der Shimeinstellungen, welche zur Ansteuerung der Shimeinheit eingesetzt werden, zumindest teilweise kompensiert werden. Das Modifizieren der Gradientenpulsform kann für diesen Anwendungsfall besonders vorteilhaft die Bildung der Ableitung der Gradientenpulsform umfassen. Das vorgeschlagene Vorgehen kann besonders vorteilhaft zum Unterdrücken der Wirbelstrombeiträge zweiter Ordnung eingesetzt werden. Gerade in Randbereichen des Aufnahmevolumens kann so eine deutliche Verbesserung der Bildqualität erreicht werden, da die Wirbelstromfelder der zweiten Ordnung typischerweise proportional zu einem Radius im Quadrat typischerweise bei hohen radiale Positionen im Aufnahmevolumen die Magnetresonanz-Bilddaten beeinflussen. Derart kann mittels der vorgeschlagenen Kompensation besonders vorteilhaft ein großes Aufnahmevolumen ohne einen Verlust der Bildqualität für die Akquisition der Magnetresonanz-Bilddaten eingesetzt werden. Beispielsweise im Fall der Schulterbildgebung kann dies besonders vorteilhaft sein. Besonders vorteilhaft können nicht nur typische ganzzahlige Terme im Wirbelstromfeld zweiter Ordnung kompensiert werden, wie beispielsweise Feldterme entlang der z-Achse (A(2,0) Terme), entlang der x-Achse (A(2,1) Terme) oder entlang der y-Achse (B(2,1) Terme), sondern auch zusätzliche Kreuzterme, wie beispielsweise B(2,2) Terme.

Eine Ausführungsform sieht vor, dass die Shimeinheit unter Verwendung der Shimeinstellungen ausschließlich dann angesteuert wird, während steigende und/oder fallende Flanken der Gradientenpulsform mittels der Gradientenspuleneinheit ausgespielt werden. Derart sind die unter Verwendung der modifizierten Gradientenpulsform generierten Shimeinstellungen insbesondere so ausgebildet, dass die von den Shimeinstellungen festgelegten Shimströme nur während des Ausspielens der steigenden und/oder fallenden Flanken durch die Shimspulen fließen. Diese vorgeschlagene Ansteuerung der Shimeinheit kann besonders vorteilhaft zur Kompensation von Wirbelstromfeldern, welche insbesondere während des Ausspielens der steigenden oder fallenden Flanken der Gradientenpulsform auftreten, eingesetzt werden. Selbstverständlich können auch weitere, nicht auf der Gradientenpulsform basierende Shimeinstellungen vorliegen, welche das Fließen von Shimströmen während konstanter Phasen der Gradientenpulsform auslösen.

Eine Ausführungsform sieht vor, dass vor der Magnetresonanz-Bildgebung des Untersuchungsobjekts in einer Kalibrierung eine Übertragungsfunktion, welche Wirbelstrombeiträge der Gradientenspule der zweiten Ordnung charakterisiert, bestimmt wird, wobei das Erzeugen der Shimeinstellungen unter Verwendung der Übertragungsfunktion erfolgt. In der Kalibrierung können dabei nicht nur die Wirbelstromfelder, auch Wirbelstrombeiträge genannt, nullter und erster Ordnung gemessen werden, sondern auch zusätzlich die Wirbelstromfelder zweiter Ordnung. Die Kalibrierung kann dabei beim Installieren eines Magnetresonanzgeräts spezifisch für dieses Magnetresonanzgerät oder spezifisch für eine Serie von Magnetresonanzgeräten durchgeführt werden. Das Bestimmen der Übertragungsfunktion kann dabei das Bestimmen von Amplituden und Zeitkonstanten für alle Kombinationen von Termen zweiter Ordnung der Wirbelstrombeiträge umfassen.

Die erfindungsgemäße Steuereinheit für ein Magnetresonanzgerät umfasst eine Gradientensteuereinheit, eine Shimsteuereinheit und eine Schnittstelle von der Gradientensteuereinheit zur Shimsteuereinheit, wobei die Schnittstelle zum Empfangen einer Gradientenpulsform von der Gradientensteuereinheit, zum Modifizieren der Gradientenpulsform und zum Übertragen der modifizierten Gradientenpulsform an die Shimsteuereinheit ausgebildet ist. Die Schnittstelle kann eine elektronische Verbindung bzw. ein Interface zwischen der Gradientensteuereinheit und der Shimsteuereinheit darstellen. Derart kann die Schnittstelle eine Eingangskomponente aufweisen, welche die Gradientenpulsform von der Gradientensteuereinheit empfängt. Die Schnittstelle kann eine Modifikationskomponente aufweisen, welche die Gradientenpulsform modifiziert. Schließlich kann die Schnittstelle eine Ausgangskomponente aufweisen, welche die modifizierte Gradientenpulsform an die Shimsteuereinheit überträgt. Die Schnittstelle kann derart ein automatisches Ansteuern der Shimeinheit, welches von dem Bereitstellen einer Gradientenpulsform von der Gradientensteuereinheit ausgelöst wird, ermöglichen. Derart kann die erfindungsgemäße Steuereinheit die bereits beschriebene automatische Kompensation der Wirbelstromfelder, welcher durch das Ausspielen der Gradientenpulsform generiert werden, ermöglichen. Auch kann die vorgeschlagene Shimeinheit gleichzeitig besonders vorteilhaft zum dem Fachmann bekannten dynamischen Shimmens eingesetzt werden, bei welchem Inhomogenitäten, insbesondere der zweiten Ordnung, auf einer schichtweisen Basis oder Atmungseinflüsse oder andere dynamische Störungen in Echtzeit kompensiert werden können. Eine Shimeinstellzeit für statische Shimeinstellungen kann für zeitkritische Prozeduren ebenfalls reduziert werden.

Eine Ausführungsform der Steuereinheit sieht vor, dass die Schnittstelle zum Bildung der Ableitung der empfangenen Gradientenpulsform und zum Übertragen der Ableitung der Gradientenpulsform an die Shimsteuereinheit ausgebildet ist. Dafür kann die Schnittstelle geeignete Komponenten, wie beispielsweise Hochpassfilter und/oder digitale Filter umfassen. Die Schnittstelle kann derart die Gleichstromkomponenten der Gradientenpulsform unterdrücken und die steigenden und/oder fallenden Flanken der Gradientenpulsform an die Shimsteuereinheit weiterleiten. Derart kann besonders vorteilhaft eine Kompensation der Wirbelströme, welche insbesondere während des Ausspielens der steigenden und fallenden Flanken der Gradientenpulsform verursacht werden, erfolgen.

Eine Ausführungsform der Steuereinheit sieht vor, dass die Schnittstelle Steuerkomponenten umfasst, welche die Übertragung der modifizierten Gradientenpulsform an die Shimsteuereinheit in Echtzeit während der Ansteuerung der Gradientenspuleneinheit mittels der Gradientensteuereinheit ermöglichen. Derart kann die Shimsteuereinheit mit der gleichen Geschwindigkeit mit der modifizierten Gradientenpulsform angesteuert werden, wie die Gradientenspuleneinheit mit der Gradientenpulsform von der Gradientensteuereinheit angesteuert wird. Derart kann die Shimsteuereinheit bzw. die Shimeinheit eine besonders vorteilhafte Kompensation der Wirbelströme, welche beim Betrieb der Gradientenspuleneinheit auftreten, durchführen.

Das erfindungsgemäße Magnetresonanzgerät umfasst eine Gradientenspuleneinheit, eine Shimeinheit und eine erfindungsgemäße Steuereinheit, wobei die Gradientensteuereinheit zum Ansteuern der Gradientenspuleneinheit mittels der Gradientenpulsform ausgebildet ist und die Shimsteuereinheit zum Erzeugen von Shimeinstellungen unter Verwendung der modifizierten Gradientenpulsform und zum Ansteuern der Shimeinheit mittels der Shimeinstellungen ausgebildet ist. Das Magnetresonanzgerät kann derart bei seinem Betrieb besonders vorteilhaft reduzierte Wirbelströme in leitenden Bauteilen des Magnetresonanzgeräts aufweisen.

Eine Ausführungsform des Magnetresonanzgeräts sieht vor, dass die Gradientensteuereinheit und die Shimsteuereinheit derart aufeinander abgestimmt sind, dass das Ansteuern der Shimeinheit während eines Ansteuerns der Gradientenspuleneinheit mittels der Gradientenpulsform erfolgt. Die zeitliche Abstimmung der Shimsteuereinheit und der Gradientensteuereinheit ermöglichen besonders vorteilhaft die Kompensation der Wirbelströme, welche durch den Betrieb der Gradientenspuleneinheit verursacht werden, durch die Shimeinheit.

Eine Ausführungsform des Magnetresonanzgeräts sieht vor, dass die Shimeinheit Shimspulen einer zweiten Ordnung aufweist, wobei die Shimspulen der zweiten Ordnung unter Verwendung der Shimeinstellungen angesteuert werden. Die Shimspulen der zweiten Ordnung können dabei besonders vorteilhaft zum Kompensieren von Wirbelstromfeldern der zweiten Ordnung eingesetzt werden.

Eine Ausführungsform des Magnetresonanzgeräts sieht vor, dass die Shimeinheit Shimspulen einer höheren Ordnung als der zweiten Ordnung aufweist, wobei die Shimspulen der höheren Ordnung als der zweiten Ordnung unter Verwendung der Shimeinstellungen angesteuert werden. Genauso wie im vorhergehenden Abschnitt beschrieben kann auch die Übertragungsfunktion für die höheren Ordnungen, beispielsweise eine dritte oder vierte Ordnung, bestimmt werden. Die Shimspulen höherer Ordnung können dann insbesondere zur Kompensation von Wirbelstromfeldern höherer Ordnung, welche durch das Ausspielen der Gradientenpulsform generiert werden, vorgesehen sein. Die Shimspulen höherer Ordnung können dabei vorteilhafterweise in Lokalspulen, welche direkt auf einem Körper des Untersuchungsobjekts platziert sind, integriert sein. Gerade bei dem Einsatz einer asymmetrischen Gradientenspuleneinheit zum Ausspielen der Gradientenpulsform kann das Kompensieren der Wirbelstromfelder höherer Ordnung besonders vorteilhaft sein.

Eine Ausführungsform des Magnetresonanzgeräts sieht vor, dass das Magnetresonanzgeräts zum Ausführen eines erfindungsgemäßen Verfahrens ausgebildet ist. Damit ist das Magnetresonanzgerät zum Ausführen eines Verfahrens zum Ansteuern einer Shimeinheit eines Magnetresonanzgeräts während einer Magnetresonanz-Bildgebung eines Untersuchungsobjekts ausgebildet. Die Gradientensteuereinheit der Steuereinheit des Magnetresonanzgeräts ist zum Bereitstellen einer Gradientenpulsform gemäß Vorgaben einer Magnetresonanz-Sequenz, welche zur Magnetresonanz-Bildgebung des Untersuchungsobjekts eingesetzt wird, ausgebildet. Die Schnittstelle ist zum Modifizieren der Gradientenpulsform ausgebildet. Die Shimsteuereinheit ist zum Erzeugen von Shimeinstellungen unter Verwendung der modifizierten Gradientenpulsform ausgebildet. Die Shimsteuereinheit ist zum Ansteuern der Shimeinheit unter Verwendung der Shimeinstellungen, wobei das Ansteuern der Shimeinheit während eines Ausspielens der Gradientenpulsform mittels der Gradientenspuleneinheit des Magnetresonanzgeräts erfolgt.

Die Vorteile der erfindungsgemäßen Steuereinheit und des erfindungsgemäßen Magnetresonanzgeräts entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
- Fig. 1: ein erfindungsgemäßes Magnetresonanzgerät mit einer erfindungsgemäßen Steuereinheit in einer schemati-schen Darstellung,
- Fig. 2: ein Ablaufdiagramm einer Ausführungsform eines er-findungsgemäßen Verfahrens und
- Fig. 3: eine exemplarische Illustration des erfindungsgemä-ßen Vorgehens.

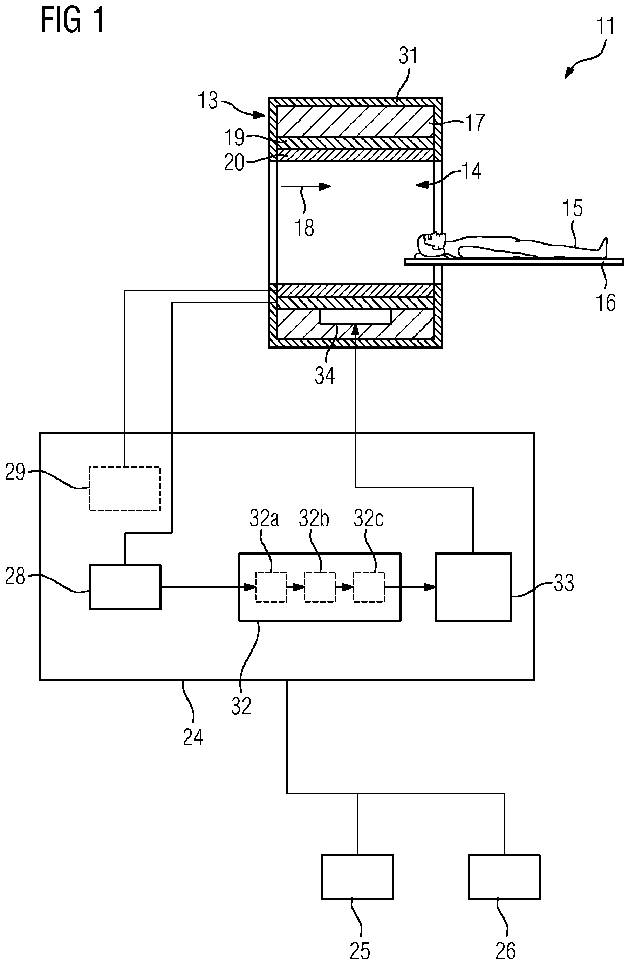

**Fig. 1** stellt ein erfindungsgemäßes Magnetresonanzgerät 11 mit einer erfindungsgemäßen Steuereinheit 24 schematisch dar.

Das Magnetresonanzgerät 11 umfasst eine von einer Magneteinheit 13 gebildete Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Untersuchungsobjekts 15, im vorliegenden Fall eines Patienten, auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Liegentisch auf, der bewegbar innerhalb des Magnetresonanzgeräts 11 angeordnet ist. Die Magneteinheit 13 ist mittels einer Gehäuseverkleidung 31 des Magnetresonanzgeräts nach außen hin abgeschirmt.

Die Magneteinheit 13 weist weiterhin eine Gradientenspuleneinheit 19 zu einer Erzeugung von Magnetfeldgradienten auf, die für eine Ortskodierung während einer Bildgebung verwendet werden. Des Weiteren weist die Magneteinheit 13 eine Hochfrequenzantenneneinheit 20, welche im gezeigten Fall als fest in das Magnetresonanzgerät 10 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 strahlt hochfrequente Magnetresonanz-Sequenzen in einen Untersuchungsraum, der im Wesentlichen von dem Patientenaufnahmebereich 14 gebildet ist, ein. Die Hochfrequenzantenneneinheit 20 ist weiterhin zum Empfang von Magnetresonanz-Signalen, insbesondere aus dem Patienten 15, ausgebildet.

Weiterhin umfasst das Magnetresonanzgerät 11 eine Shimeinheit 34. Die in Fig. 1 gezeigte Shimeinheit 34 ist in einer unmittelbaren räumlichen Umgebung der Gradientenspuleneinheit 19 bzw. integriert in die Gradientenspuleneinheit 19 angeordnet. Es ist aber auch denkbar, dass Teile der Shimeinheit 34 oder die gesamte Shimeinheit 34 in nicht dargestellten Lokalspulen des Magnetresonanzgeräts 11 angeordnet ist. Die Shimeinheit kann mehrere Shimkanäle bzw. Shimspulen umfassen. Die Shimeinheit 34 weist insbesondere Shimspulen einer zweiten Ordnung aufweist, wobei die Shimspulen der zweiten Ordnung unter Verwendung von Shimeinstellungen angesteuert werden. Die Shimeinheit 34 kann auch Shimspulen einer höheren Ordnung als der zweiten Ordnung aufweisen, wobei die Shimspulen der höheren Ordnung als der zweiten Ordnung unter Verwendung der Shimeinstellungen angesteuert werden.

Die Steuereinheit 24 umfasst eine Gradientensteuereinheit 28 und eine Shimsteuereinheit 33. Die Gradientensteuereinheit 28 ist zum Ansteuern der Gradientenspuleneinheit 19 mittels einer Gradientenpulsform ausgebildet. Die Shimsteuereinheit 33 ist zum Erzeugen von Shimeinstellungen und zum Ansteuern der Shimeinheit 34 mittels der Shimeinstellungen ausgebildet ist. Die dargestellte Steuereinheit 24 umfasst im gezeigten Fall auch eine Hochfrequenzantennensteuereinheit 29, welche zur Ansteuerung der Hochfrequenzantenneneinheit 20 ausgebildet ist.

Weiterhin umfasst die Steuereinheit 24 eine Schnittstelle 32 von der Gradientensteuereinheit 28 zur Shimsteuereinheit 33. Die Schnittstelle umfasst eine Eingangskomponente 32a, welche eine Gradientenpulsform von der Gradientensteuereinheit 28 empfängt. Die Schnittstelle umfasst Modifikationskomponente 32b, welche die Gradientenpulsform modifiziert. Die Schnittstelle weist eine Ausgangskomponente 32c auf, welche die modifizierte Gradientenpulsform an die Shimsteuereinheit 33 überträgt. Die Shimsteuereinheit 33 kann dann die Shimeinstellungen unter Verwendung der modifizierten Gradientenpulsform erzeugen.

Vorteilhafterweise kann die Schnittstelle 32, insbesondere die Modifikationskomponente 32b und die Ausgangskomponente 32c der Schnittstelle 32, zum Bildung der Ableitung der empfangenen Gradientenpulsform und zum Übertragen der Ableitung der Gradientenpulsform an die Shimsteuereinheit 33 ausgebildet sein. Vorteilhafterweise kann die Schnittstelle bzw. Steuerkomponenten der Schnittstelle, wie beispielsweise die Ausgangskomponente 32c, die Übertragung der modifizierten Gradientenpulsform an die Shimsteuereinheit 32c in Echtzeit während der Ansteuerung der Gradientenspuleneinheit 19 mittels der Gradientensteuereinheit 28 ermöglichen. Vorteilhafterweise sind die Gradientensteuereinheit 28 und die Shimsteuereinheit 33 derart aufeinander abgestimmt, dass das Ansteuern der Shimeinheit 34 während eines Ansteuerns der Gradientenspuleneinheit 19 mittels der Gradientenpulsform erfolgt.

Rekonstruierte Magnetresonanz-Bilder können auf einer Bereitstellungseinheit 25, im vorliegenden Fall einer Anzeigeeinheit 25, des Magnetresonanzgeräts 11 für einen Benutzer bereitgestellt werden. Zudem weist das Magnetresonanzgerät 11 eine Eingabeeinheit 26 auf, mittels derer Informationen und/oder Parameter während eines Messvorgangs von einem Benutzer eingegeben werden können.

Das Magnetresonanzgerät 11 ist somit zusammen mit der Steuereinheit 24 zur Ausführung eines erfindungsgemäßen Verfahrens zum Ansteuern der Shimeinheit 34 während einer Magnetresonanz-Bildgebung des Untersuchungsobjekts 15 ausgelegt.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

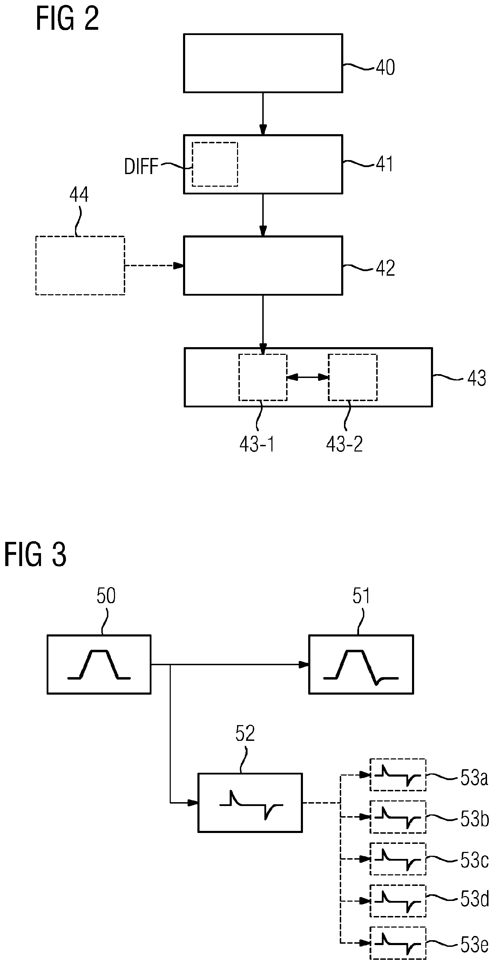

**Fig. 2** zeigt ein Ablaufdiagramm einer ersten Ausführungsform eines erfindungsgemäßen Verfahrens zum Ansteuern der Shimeinheit 34 des Magnetresonanzgeräts 11 während einer Magnetresonanz-Bildgebung des Untersuchungsobjekts 15.

In einem ersten Verfahrensschritt 40 erfolgt ein Bereitstellen einer Gradientenpulsform mittels der Gradientensteuereinheit 28 der Steuereinheit 24 gemäß Vorgaben einer Magnetresonanz-Sequenz, welche zur Magnetresonanz-Bildgebung des Untersuchungsobjekts 15 eingesetzt wird. Die Gradientenpulsform wird anschließend von der Gradientensteuereinheit 28 zur Schnittstelle 32 der Steuereinheit 24 übertragen.

In einem weiteren Verfahrensschritt 41 erfolgt ein Modifizieren der Gradientenpulsform mittels der Schnittstelle 32 der Steuereinheit 24. Das Modifizieren der Gradientenpulsform umfasst dabei insbesondere eine Bildung einer Ableitung der Gradientenpulsform in einem Teilschritt DIFF des weiteren Verfahrensschritts 41. Die Ableitung der Gradientenpulsform kann dann von der Schnittstelle 32 zur Shimsteuereinheit 33 übertragen werden.

In einem weiteren Verfahrensschritt 42 erfolgt ein Erzeugen von Shimeinstellungen unter Verwendung der modifizierten Gradientenpulsform mittels der Shimsteuereinheit 33 der Steuereinheit 24. Das Erzeugen der Shimeinstellungen erfolgt dabei insbesondere unter Verwendung der Ableitung der Gradientenpulsform, welche im Teilschritt DIFF des weiteren Verfahrensschritts 41 erzeugt worden ist.

In einem weiteren Verfahrensschritt 43 erfolgt ein Ansteuern der Shimeinheit 34 unter Verwendung der Shimeinstellungen mittels der Shimsteuereinheit 33 in einem ersten Teilschritt 43-1 des weiteren Verfahrensschritts 43. Gleichzeitig erfolgt ein Ausspielen der Gradientenpulsform mittels der Gradientenspuleneinheit 19 des Magnetresonanzgeräts 11 in einem zweiten Teilschritt 43-2 des weiteren Verfahrensschritts 43. Es wird dabei insbesondere die originale Gradientenpulsform und nicht die modifizierte Gradientenpulsform mittels der Gradientenspuleneinheit 19 ausgespielt. Das Ansteuern der Shimeinheit 34 erfolgt dabei während des Ausspielens der Gradientenpulsform mittels der Gradientenspuleneinheit 19. Besonders vorteilhaft wird die Shimeinheit 34 unter Verwendung der Shimeinstellungen ausschließlich dann angesteuert wird, während steigende und/oder fallende Flanken der Gradientenpulsform mittels der Gradientenspuleneinheit 19 ausgespielt werden.

Im Verfahren gemäß Fig. 2 erfolgen das Modifizieren der Gradientenpulsform und das Erzeugen der Shimeinstellungen unter Verwendung der modifizierten Gradientenpulsform derart, dass Wirbelstrombeiträge zweiter Ordnung, welche während des Ausspielens der Gradientenpulsform mittels der Gradientenspuleneinheit 19 auftreten, mittels der Shimeinstellungen, welche zur Ansteuerung der Shimeinheit 33 eingesetzt werden, zumindest teilweise kompensiert werden.

Vor der Magnetresonanz-Bildgebung des Untersuchungsobjekts 15 kann vorteilhafterweise in einem weiteren Verfahrensschritt 44 in einer Kalibrierung eine Übertragungsfunktion, welche Wirbelstrombeiträge der Gradientenspule 19 der zweiten Ordnung charakterisiert, bestimmt werden, wobei das Erzeugen der Shimeinstellungen unter Verwendung der Übertragungsfunktion erfolgt.

Fig. 3 zeigt eine exemplarische Illustration des erfindungsgemäßen Vorgehens. Selbstverständlich sind die in Fig. 3 dargestellten Formen nur als beispielhaft anzusehen. Die gezeigte Gradientenpulsform und ihre Modifikationen können selbstverständlich auch anders als in Fig. 3 dargestellt ausgebildet sein.

Im Block 50 ist eine exemplarische Gradientenpulsform dargestellt, welche von der Gradientensteuereinheit 28 bereitgestellt wird. Die Gradientenpulsform umfasst ein Plateau mit konstanter Amplitude, eine steigende Flanke und eine fallende Flanke.

Die Gradientenpulsform wird nun zur Gradientenspuleneinheit 19 übertragen, von welcher sie zur Magnetresonanz-Bildgebung, wie in Block 51 dargestellt, ausgespielt wird.

Die Gradientenpulsform wird nicht nur zur Gradientenspuleneinheit 19 übertragen, sondern zusätzlich auch zur Schnittstelle 32. Die Schnittstelle modifiziert in einem weiteren die Gradientenpulsform. Eine besonders vorteilhafte Modifikation der Gradientenpulsform ist dabei das Bilden der Ableitung der Gradientenpulsform. In Block 52 ist die Ableitung der in Block 50 gezeigten Gradientenpulsform dargestellt. Das Plateau mit konstanter Amplitude zeigt sich in der Ableitung der Gradientenpulsform als Nulllinie. Die steigende und die fallende Flanke der Gradientenpulsform treten dagegen in der Ableitung wie in Block 52 ersichtlich deutlich hervor.

Die modifizierte Gradientenpulsform, also insbesondere die Ableitung der Gradientenpulsform, wird nun von der Schnittstelle 32 zur Shimsteuereinheit 33 übertragen. Die Shimsteuereinheit 33 kann anhand der Ableitung der Gradientenpulsform Shimeinstellungen erzeugen, mittels welcher die Shimeinheit 34 von der Shimsteuereinheit 33 angesteuert wird.

In den Blöcken 53a, 53b, 53c, 53d, 53e sind exemplarische Stromverteilungen für fünf Shimspulen zweiter Ordnung der Shimeinheit 34 dargestellt. Die gezeigten Stromverteilungen basieren dabei auf der in Block 52 gezeigten Ableitung der Gradientenpulsform. Die Stromverteilungen sind im gezeigten Fall für alle fünf Shimspulen gleich ausgebildet, wobei dies in einem konkreten Anwendungsfall nicht der Fall sein muss.

Die Gradientenpulsform 51 kann nun von der Gradientenspuleneinheit 19 ausgespielt werden, zeitgleich während die Shimspulen der Shimeinheit 34 mit den in den Blöcken 53a, 53b, 53c, 53d, 53e gezeigten Stromverteilungen belegt werden. Es ist ersichtlich, dass lediglich dann Strom durch die Shimspulen fließt, wenn die steigende und die fallende Flanke der Gradientenpulsform von der Gradientenspuleneinheit 19 ausgespielt werden. Derart ist eine besonders vorteilhafte Kompensation der Wirbelstromfelder, welche durch das Ausspielen der steigenden und die fallenden Flanke der Gradientenpulsform erzeugt werden, durch die in den Blöcken 53a, 53b, 53c, 53d, 53e gezeigten Stromverteilungen in den Shimspulen der zweiten Ordnung möglich.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Ansteuern einer Shimeinheit eines Magnetresonanzgeräts während einer Magnetresonanz-Bildgebung eines Untersuchungsobjekts, umfassend folgende Verfahrensschritte:
- Bereitstellen einer Gradientenpulsform gemäß Vorgaben einer Magnetresonanz-Sequenz, welche zur Magnetresonanz-Bildgebung des Untersuchungsobjekts eingesetzt wird,
- Modifizieren der Gradientenpulsform,
- Erzeugen von Shimeinstellungen unter Verwendung der modifizierten Gradientenpulsform,
- Ansteuern der Shimeinheit unter Verwendung der Shimeinstellungen, wobei das Ansteuern der Shimeinheit während eines Ausspielens der Gradientenpulsform mittels einer Gradientenspuleneinheit des Magnetresonanzgeräts erfolgt.

2. Verfahren nach Anspruch 1, wobei das Modifizieren der Gradientenpulsform eine Bildung der Ableitung der Gradientenpulsform umfasst, wobei das Erzeugen der Shimeinstellungen unter Verwendung der Ableitung der Gradientenpulsform erfolgt.

3. Verfahren nach einem der vorhergehenden Ansprüche, wobei das Modifizieren der Gradientenpulsform und das Erzeugen der Shimeinstellungen unter Verwendung der modifizierten Gradientenpulsform derart erfolgen, dass Wirbelstrombeiträge zweiter Ordnung, welche während des Ausspielens der Gradientenpulsform mittels der Gradientenspuleneinheit auftreten, mittels der Shimeinstellungen, welche zur Ansteuerung der Shimeinheit eingesetzt werden, zumindest teilweise kompensiert werden.

4. Verfahren nach einem der vorhergehenden Ansprüche, wobei die Shimeinheit unter Verwendung der Shimeinstellungen ausschließlich dann angesteuert wird, während steigende und/oder fallende Flanken der Gradientenpulsform mittels der Gradientenspuleneinheit ausgespielt werden.

5. Verfahren nach einem der vorhergehenden Ansprüche, wobei vor der Magnetresonanz-Bildgebung des Untersuchungsobjekts in einer Kalibrierung eine Übertragungsfunktion, welche Wirbelstrombeiträge der Gradientenspule der zweiten Ordnung charakterisiert, bestimmt wird, wobei das Erzeugen der Shimeinstellungen unter Verwendung der Übertragungsfunktion erfolgt.

6. Steuereinheit für ein Magnetresonanzgerät, umfassend eine Gradientensteuereinheit, eine Shimsteuereinheit und eine Schnittstelle von der Gradientensteuereinheit zur Shimsteuereinheit, wobei die Schnittstelle zum Empfangen einer Gradientenpulsform von der Gradientensteuereinheit, zum Modifizieren der Gradientenpulsform und zum Übertragen der modifizierten Gradientenpulsform an die Shimsteuereinheit ausgebildet ist.

7. Steuereinheit nach Anspruch 6, wobei die Schnittstelle zum Bildung der Ableitung der empfangenen Gradientenpulsform und zum Übertragen der Ableitung der Gradientenpulsform an die Shimsteuereinheit ausgebildet ist.

8. Steuereinheit nach einem der Ansprüche 6-7, wobei die Schnittstelle Steuerkomponenten umfasst, welche die Übertragung der modifizierten Gradientenpulsform an die Shimsteuereinheit in Echtzeit während der Ansteuerung der Gradientenspuleneinheit mittels der Gradientensteuereinheit ermöglichen.

9. Magnetresonanzgerät, umfassend eine Gradientenspuleneinheit, eine Shimeinheit und eine Steuereinheit nach einem der Ansprüche 6-8, wobei die Gradientensteuereinheit zum Ansteuern der Gradientenspuleneinheit mittels der Gradientenpulsform ausgebildet ist und die Shimsteuereinheit zum Erzeugen von Shimeinstellungen unter Verwendung der modifizierten Gradientenpulsform und zum Ansteuern der Shimeinheit mittels der Shimeinstellungen ausgebildet ist.

10. Magnetresonanzgeräts nach Anspruch 9, wobei die Gradientensteuereinheit und die Shimsteuereinheit derart aufeinander abgestimmt sind, dass das Ansteuern der Shimeinheit während eines Ansteuerns der Gradientenspuleneinheit mittels der Gradientenpulsform erfolgt.

11. Magnetresonanzgerät nach einem der Ansprüche 9-10, wobei die Shimeinheit Shimspulen einer zweiten Ordnung aufweist, wobei die Shimspulen der zweiten Ordnung unter Verwendung der Shimeinstellungen angesteuert werden.

12. Magnetresonanzgerät nach einem der Ansprüche 9-11, wobei die Shimeinheit Shimspulen einer höheren Ordnung als der zweiten Ordnung aufweist, wobei die Shimspulen der höheren Ordnung als der zweiten Ordnung unter Verwendung der Shimeinstellungen angesteuert werden.

13. Magnetresonanzgerät nach einem der Ansprüche 9-12, wobei das Magnetresonanzgeräts zum Ausführen eines Verfahrens nach einem der Ansprüche 1-5 ausgebildet ist.
